# EUROPEAN PATENT SPECIFICATION

(11) **EP 1 837 655 B1**
(45) Date of publication and mention of the grant of the patent: **22.09.2010**
(21) Application number: 05819496.0
(22) Date of filing: 26.12.2005
(51) Int. Cl.: G01N 33/535

(54) **BLOCKED ENZYME PROBE COMPLEX**
BLOCKIERTER ENZYMSONDENKOMPLEX
COMPLEXE DE SONDE ENZYMATIQUE BLOQUE

(30) Priority: 28.12.2004 JP 2004378906
(43) Date of publication of application: 26.09.2007
(73) Proprietor: Advanced Life Science Institute, Inc., Wako-shi, Saitama, 351-0112 (JP)
(72) Inventor: AOYAGI, Katsumi, Wako-shi, Saitama 351-0112 (JP)
(74) Representative: Merkle, Gebhard
(86) International application number: PCT/JP2005/023763
(87) International publication number: WO 2006/070732

(56) References cited:
- EP-A- 0 175 560
- EP-A- 0 270 930
- WO-A1-95/02824
- JP-A- 2000 088 850
- JP-A- 2001 013 145
- JP-A- 2001 181 299
- JP-A- 2003 194 821
- FUENTES MANUEL ET AL: "Determination of protein-protein interactions through aldehyde-dextran intermolecular cross-linking" PROTEOMICS, vol. 4, no. 9, September 2004 (2004-09), pages 2602-2607, XP002489780 ISSN: 1615-9853
- Varun Shastri: "Academic Dictionary of Bio-chemistry" 2005, Isha Books , Delhi ISBN: 82-8205-186-X , page 215

## Description

### Technical Field

The present invention relates to technology for labeling a probe with an enzyme. A blocked enzyme-probe complex thus produced is widely used in immunoassays using an immuno-reaction such as enzyme immunoassay, immunohistochemistry and the like.

### Background Art

Enzyme-labeled probes that are probes labeled with enzymes have been widely used in general in immunological detection or assay methods. For example, probes labeled with horseradish peroxidase (HRP), alkaline phosphatase (ALP), β-galactosidase, glucose oxidase and the like can be used in the detection step in immunohistochemistry and enzyme immunoassay.

Immunohistochemistry and enzyme immunoassays have been used widely for a long time as methods for detecting self antigens or foreign antigens in the living body. Since these detection methods using immuno-reactions have high specificity and sensitivity, a minute amount of substances in the body can be detected without isolating them. However, there are many substances present in the body in such a minute amount that general immunohistochemistry and enzyme immunoassay cannot detect, and studies have been conducted to find the methods for increasing the sensitivity of the assay methods to detect such substances.

For example, the concentration of tumor markers such as carcinoembryonic antigen (CEA) and α-fetoprotein in the serum of healthy people is 5 to 20 ng/ml. However, the concentration of human gastrin-releasing peptide precursor (ProGRP) in the serum of healthy individuals is about 14 pg/ml, and 1000 times higher sensitivity would be required to detect ProGRP. Further, for foreign antigens, for example, hepatitis C virus exists in a very small amount in the blood, and thus a highly sensitive antigen-detecting method has been desired. To detect the antigen, a sensitivity level that can detect 100 to 1000 copies of viral RNA and about 0.03 to 0.3 pg/ml protein concentration is required.

Efforts have been made to increase the sensitivity of immunoassay methods to detect antigens or substances that exist in such minute amounts in the body. Ishikawa et al., (Non-patent Document 1) have described in detail investigations to increase sensitivity of enzyme immunoassay. Factors that affect sensitivity of enzyme immunoassay include a type of assay systems, detection sensitivity of a label, a type of a labeling method, duration of an immuno-reaction, and affinity between an antigen and an antibody. Further, conditions affecting the improvement of the sensitivity in assaying antigen by the Sandwich method include: conditions for attaching antibody to a solid phase; reaction efficiency of an antigen; reaction efficiency of an enzyme-labeled antibody; reduction of non-specific absorption of a labeled antibody to a solid phase; the amount of a labeled antibody to be added; duration for immuno-reaction; temperature, pH, ionic strength, and choice of a buffer for immuno-reaction; stereochemical configuration and the number of antigenic determinant groups.

In addition to the investigations described above, for increasing sensitivity of the enzyme immunoassay, efforts have been made to improve the labeling method in regard to the cross-linkage between an enzyme and an antibody at the detection step. Various methods for preparing a labeled antibody have been known, and especially, the method for preparing a labeled antibody reported by Ishikawa et al. has been applied widely to general diagnostic reagents. In the typical method, one to several enzymes are bound to mainly an antibody (in the case of IgG, the molecular weight is about 150,000, in the case of Fab', the molecular weight is about 46,000), and, for example, the total molecular weight of a complex, in which 3 molecules of horseradish peroxidase (HRP) (molecular weight: 40,000) and one molecule of IgG are bound together, is 40,000 × 3 + 150,000 = 270,000. Also, the total molecular weight of a complex, in which 3 molecules of alkaline phosphatase (ALP) (molecular weight: 100,000) and one molecule of IgG are bound together, is 100,000 x 3 + 150,000 = 450,000. However, Ishikawa et al., provides descriptions showing that measurement with the highest sensitivity was achieved preferably by binding an antibody to an enzyme at a ratio of 1 molecule: 1 molecule, especially by using the Fab' portion after removing the Fc portion, and a method was developed to covalently link one molecule of an antibody to one enzyme molecule (Non-patent Document 2). In the case of the antibody enzyme-labeling method by which enzyme is bound to an antibody at 1:1 ratio, the total molecular weight of a complex of, for example, 1 molecule of HRP and 1 molecule of Fab' covalently linked is 40,000 + 46,000 = 86,000. In general, enzyme-labeled complexes with a total molecular weight of 200,000 or less are mostly used.

To develop the immunoassay method highly sensitive, increasing the signal by increasing the number of steps has been tried in various manners. For example, by using the high binding ability of biotin and avidin, several molecules of biotin are introduced mainly to a secondary antibody, and after reacting the biotinated secondary antibody to a material to be analyzed, an excess amount of the biotinated secondary antibody is removed. Avidin-enzyme is then added to form a biotinated secondary antibody-avidin-enzyme complex, and the sensitivity is enhanced by increasing the number of enzymes molecules linked to the secondary antibody. It is possible to use an avidin-biotin-enzyme complex in place of avidin-enzyme. Also, Butler et al., describe an antibody-enzyme complex that is peroxidase-anti-peroxidase antibody (Non-patent Document 3). Bobrow et al., reacted peroxidase-labeled secondary antibody with a material to be analyzed, and after removing excess peroxidase-labeled secondary antibody, added biotin-Tyramide to bind radicalized biotin-Tyramide to blocking protein around peroxidase-labeled secondary antibody and, after washing, amplified enzyme signal using peroxidase-labeled streptavidin (Non-patent Document 4). However, these methods have shortcomings such as increasing the number of steps and time for measurement.

A method for binding an enzyme to a certain carrier and then binding an antibody to the enzyme-linked carrier has been reported to increase the number of enzyme molecules bound to the antibody, and according to this method the sensitivity can be increased with the minimum number of steps (Patent Document 1). In this method, maleimide groups or thiol (SH) groups are introduced to a carrier having amino groups and an enzyme is linked to the carrier. A maleimide group is introduced to at least one amino group left on the carrier to which antibody is bound. Since an antibody and an enzyme are linked via a carrier in this method, it would appear that a larger number of enzyme molecules can be linked than in the method of directly binding an enzyme to the antibody and thus the sensitivity is increased. However, the inventors mentioned that the molecular weight of the carrier is suitably 5,000 to 500,000, preferably 10,000 to 300,000. In this case, for example, when horseradish peroxidase is used as the enzyme, the molecular weight is about 40,000. Even if the molecular weight of the carrier is 500,000, it is natural that the number of molecules with 40,000 MW capable of binding to a molecule with 500,000 MW is physically and spatially limited and thus there is a limit in the number of enzyme molecules capable of binding to the carrier. That is, when the carrier is bound to an enzyme and an antibody, the functional groups on the carrier have to be shared by an enzyme and an antibody, the number of enzyme molecules is decreased and the signal would be lowered. When a larger number of enzyme molecules bound to the carrier, there would be less space for an antibody to bind, and the reactivity to an antigen would be decreased. It means that though it would be better if an antibody and an enzyme are bound to the same carrier as much as possible, depending on the preparation process for macromolecules, aggregation and precipitation tend to occur, causing higher background on measurement resulting the lower sensitivity.
Patent Document 1: Japanese Patent.Application Laying Open (KOKAI) No. 2000-88850
Non-patent Document 1: Ultra High Sensitive Enzyme Immunoassay Method, Eiji Ishikawa, 1993, Japan Scientific Societies Press
Non-patent Document 2: Imagawa et al., J. Appl. Biochem. Vol. 4;400, 1982
Non-patent Document 3: Butler, (1981) Methods Enzymol., Vol. 73;482-523
Non-patent Document 4: Bobrow, (1989) J, Immunol. Methods, 125, 279-285

The state-of-the-art document FUENTES MANUEL ET AL: PROTEOMICS, vol. 4, no. 9, September 2004, pages 2602-2607, describes a strategy to avoid non-specific protein-protein associations in a enzyme cross-linked immunocomplex, by using aldehyde dextrans to link the enzymes in such a way that application of the enzymes is prevented.

The document EP-A-0 175 560 (DU PONT) 26 March 1986 is concerned with cross-linked enzyme conjugates, which are covalently coupled to an antibody. These conjugates provide enhanced signal sensitivity in the corresponding immunoassays. The cross-linking agents are low molecular weight organic compounds with reactive groups.

The patent application EP-A-0 270 930 (MILES INC) 15 June 1988 describes a method for obtaining a substantially pure preparation of an enzyme antibody conjugate having an enzyme component covalently coupled to a predetermined number of antibody components. These conjugates are useful in labeled reagents in immunometric assays.

Reference is also made to the state-of-the-art documents: JP 2003 194821 A, JP 2000 088850 A (& EP 0992794 A), JP 2001 181299 A, JP 2001 013145 A.

### Disclosure of the Invention

### Problems to be solved by the invention

The present inventors tentatively prepared enzyme-labeled probes according to the preparation method described in the above patent application and applied them to the assay systems for ProGRP and HCV antigen for which high sensitivity is required. However, in the assay systems for measuring a minute amount of biomaterials, even using the enzyme-labeled probes, which have higher sensitivity than the conventional ones, sufficient sensitivity was not necessarily obtained. Therefore, the problem to be solved of the present invention is to provide a highly sensitive enzyme-labeled probe which can be used for high sensitivity assay systems.

### Means for solving the problem

The present inventors conducted the investigation to obtain a highly sensitive enzyme-labeled probe. As the result, the present inventors produced a blocked complex, in which two or more molecules as carrier are linked via an enzyme bound to the carrier, and successfully achieved the objective, the highly sensitive blocked enzyme-probe complex, by binding a probe to this blocked complex.

That is, the present invention is:
(1) A blocked enzyme-probe complex wherein a probe molecule is conjugated to a complex in which two or more molecules as carrier having a molecular weight of 20,000 to 4,000,000 Da are linked via an enzyme, bound to said carrier, the carrier being selected from the the group consisting of polysaccharides, high molecular weight proteins and peptide polymers wherein more enzyme molecules are bound to the complex.
(2) The blocked enzyme-probe complex according to (1) wherein the carrier and the enzyme molecule are bound through a functional group on the carrier and an aldehyde group formed by oxidizing a carbohydrate chain within the enzyme molecule.
(3) The blocked enzyme-probe complex according to (1) or (2) wherein the carrier is one or more varieties selected from the group consisting of dextran, aminodextran, ficoll, dextrin, agarose, pullulan, various celluloses, chitin, chitosan, β-galactosidase, thyroglobulin, hemocyanin, polylysine, polypeptide and DNA.
(4) The blocked enzyme-probe complex according to (1) or (2) wherein the enzyme is one or more varieties selected from the group consisting of horseradish peroxidase, alkaline phosphatase, β-galactosidase, glucose oxidase and luciferase
(5) The blocked enzyme-probe complex according to (1) or (2) wherein the probe is one or more varieties selected from the group consisting of an antibody molecule or a functional fragment thereof, protein A, protein G, protein L, lectin, a receptor and avidin.
(6) The blocked enzyme-probe complex according to any one of (1)-(5) wherein two or more varieties of probes are conjugated.
(7) The blocked enzyme-probe complex according to (5) or (6) wherein the antibody molecule or the functional fragment thereof is one or more varieties selected from the group consisting of anti-HCV core antigen antibody, anti-gastrin releasing peptide precursor antibody and the functional fragments thereof.
(8) The blocked enzyme-probe complex according to any one of (1)-(7) wherein the molecular weight of the blocked enzyme-probe complex is 440,000 Da or more.
(9) An immunoassay kit or a nucleic acid detection reagent comprising the blocked enzyme-probe complex according to any one of (1)-(8).
(10) A method for producing the blocked enzyme-probe complex according to any one of (1)-(8) comprising the steps of forming a blocked material by binding a carrier having a molecular weight of 20,000 to 4,000,000 Da to an enzyme; and conjugating a probe to the blocked material.
(11) The method according to (10) wherein the blocked material is formed by reacting the carrier and the enzyme molecule at the weight ratio of carrier: enzyme = 1:0.1 to 1:20.

Further, the linking of two or more molecules as carrier having a molecular weight of 20,000 to 4,000,000 according to (1) of the present invention includes linking not directly mediated by an enzyme. For example, it is possible to be mediated by a linker having a functional group or by a protein. Furthermore, the present invention encompasses a blocked enzyme-probe complex in which a carrier, an enzyme and a probe are respectively bound via a linker molecule having a functional group. That is, the present invention is a blocked enzyme-probe complex, in which carriers are bound to each other via enzymes or linkers, and enzymes and probes are bound to the molecule, whereby it has many enzyme or probe molecules in one molecule.

The blocked enzyme-probe complex of the present invention can be produced by binding an enzyme to a carrier to form a blocked material and by binding a probe molecule to this blocked material. The blocked enzyme-probe complex of the present invention can also be produced by binding an enzyme and a probe to a carrier to form a conjugate and then linking the conjugate each other to form a blocked complex.

In the present invention, a larger number of enzyme molecules or probes is bound to the blocked material by increasing the molecular weight by linking carrier molecules through an enzyme. On the other hand, since the probes are bound to the surface of the blocked material, steric hindrance hardly occurs in the blocked enzyme-probe complex, and thus the complex can be produced regardless of the probe moleculer size. For the final product of the blocked enzyme-probe complex, the blocked enzyme-probe complex having a molecular weight of 440,000 or above has a high efficacy and further, one having a molecular weight of 668,000 or above has a higher sensitivity. In principle, the greater the molecular weight of the blocked enzyme-probe complex becomes, the more enzyme molecules to one complex molecule is bound, and the greater the sensitivity becomes. The blocked enzyme-probe complex having a large molecular weight can be produced by the method according to the present invention, and methods such as gel filtration and the like can be used to select the blocked enzyme-probe complex having a larger molecular weight.

Although the molecular weight after the blocked complex formation may be a little variable depending on the carrier, enzyme and probe to be used, any molecular weight may be acceptable as long as the blocked enzyme-probe complex does not precipitate or sediment in liquid and no upper limit of the molecular weight should be set. For the reference of the molecular weight, 20,000,000 has no problem at all when Dextran is used as the carrier (Examples 1 and 6 described below); and there is no problem of the molecular weight between 40,000,000 and 100,000,000.

The present invention was achieved originally while searching for an enzyme-probe complex which can be used in the field of immunoassay and immunohistochemistry, but there is no limit in applying the complex to other fields.

### Advantageous effect of the invention

The enzyme-labeled antibody of the present invention makes detection of antigen and protein possible, which are present in the living body in a trace amount and by a conventional enzyme-labeled antibody could be not detected. Using this enzyme-labeled antibody, antigens and proteins that could not be assayed before can be assayed.

### Brief Description of the Drawings

Figure 1 shows the result of molecular weight analysis of the enzyme-anti-HCV core antigen monoclonal antibody complex of the present invention by gel filtration;
Figure 2 shows the result of comparison of detection sensitivity for core antigen using the enzyme-anti-HCV core antigen monoclonal antibody complex and conventional enzyme-antibody;
Figure 3 shows the reactivity when one kind of monoclonal antibody was bound to the carrier-enzyme complex and when two kinds of monoclonal antibody were bound to the carrier-enzyme complex;
Figure 4 shows the reactivity of the complex in which amino groups in an enzyme are blocked and then bound to a probe and the complex in which amino groups in an enzyme are not blocked and bound to a probe; and
Figure 5 shows the result of a comparison of detection sensitivity for ProGRP using the enzyme-anti-ProGRP antigen monoclonal antibody complex and the enzyme-antibody by the conventional method.

### Best Mode for Carrying Out the Invention

The carrier in the present invention is not particularly limited as long as the molecular weight is in the range of 20,000 to 4,000,000. and the carrier is selected from the group consisting of polysaccharides, high molecular weight proteins and peptide polymers. However, since it is desirable that a large number of enzyme molecules are bound in order to increase the sensitivity, the molecular weight at a certain level is preferred. Examples of the carrier include polysaccharides, high molecular weight proteins and peptide polymers, and the suitable molecular weight thereof is in the range of 20,000 to 20,000,000, preferably 20,000 to 4,000,000 and more preferably 70,000 to 2,000,000. Further, when polysaccharides or peptide polymers are used, the signal tends to be stronger for molecules rich in side chains even if the molecular weight is the same.

Examples of the polysaccharide carrier of the present invention include dextran, aminodextran, ficoll, dextrin, agarose, pullulan, various celluloses, chitin, chitosan, soluble starch and the like. Further, examples of the high molecular weight protein carrier of the present invention include β-galactosidase, thyroglobulin, hemocyanin and the like. Still further, polylysine as well as various peptide polymers can be used as the peptide polymer carrier of the present invention.

The enzyme that can be used in the present invention is not particularly limited, and horseradish peroxidase (HRP), alkaline phosphatase (ALP), β-galactosidase, glucose oxidase, luciferase and the like, which are generally in use for immunoassay, are suitably used. Since the enzyme binds to two or more carriers, or carrier and probe molecules, it is desirable that the enzyme contains two or more functional groups, for example, a carbohydrate chain and an amino group; or two or more amino groups; an amino group and a carboxyl group; and a thiol group and an amino group.

Any linker or binding mode may be used to bind the carrier to the enzyme. However, since it is necessary to bind the probes to the blocked material after binding the carrier to the enzyme, functional groups must be left on the enzyme or the carrier.

A blocked material mediated by enzyme molecules can be produced by binding enzyme molecules to a carrier having, for example, a molecular weight in the range of 20,000 to 4,000,000 through hydrazine groups, which are present on the carrier or introduced to the carrier using appropriate linker molecules, or which are introduced to the enzyme molecules using appropriate linker molecules. The blocked enzyme-probe complex can be produced by binding probe molecules through functional groups in the carrier molecules or in the enzyme molecules which are linked to the carrier in the blocked material.

Similarly, the blocked enzyme-probe complex can be produced by binding hydrazine groups introduced to a carrier having a molecular weight in the range of 20,000 to 4,000,000 to aldehyde groups produced by oxidizing a carbohydrate chain in an enzyme molecule to bind the carrier to the enzyme molecule, and subsequently binding probe molecules via linker molecules bound to functional groups in the carrier molecule or in the enzyme molecules bound to the carrier in the blocked material formed via the enzyme molecules.

In this case, the linker molecule having a hydrazine group, which is introduced to the carrier or enzyme, may be a hydrazine salt such as hydrazine sulfate and hydrazine hydrochloride having a hydrazine group (-NHNH₂), a hydrazide having a hydrazine group (-CO-NHNH2) or a substance having a functional group and a hydrazine group.

Also, a substance having a functional group such as a maleimide group, succimidyl group, carboxyl group, thiol group and the like can be used as a linker molecule which links the carrier or the enzyme molecule to the probe molecules.

Further, when preparing the blocked enzyme mediated by an enzyme, the ratio of the weight of an enzyme to a carrier is preferably 0.2 to 10 fold, and more preferably 0.3 to 5 fold, although it is dependent on the number of functional groups, such as hydrazine groups, on the carrier.

As an another example, a blocked enzyme mediated by enzyme is prepared by oxidizing carbohydrate chains of horseradish peroxidase (HRP), which is in turn bound to Dextran with a molecular weight of 20,000 or more, to which hydrazine groups are introduced. To introduce maleimide groups to the surface of the blocked enzyme, linker molecules such as N-(6-maleimidecaproyloxy)succinimide (EMCS) are linked to residual hydrazine groups on the carrier and residual amino groups on HRP and reacted to SH groups present in or introduced to the probe molecule to prepare the blocked enzyme-probe complex. In this way, carbohydrate chains in the enzyme molecule, and residual hydrazine groups and residual amino groups are utilized to prepare the blocked enzyme-probe complex.

Any cross-linking agent can be used to bind the blocked material and the probe, and any binding mode can be used. Heterobifunctional crosslinker or homobifunctional crosslinker which is highly stable in an aqueous solution is preferably used.

Antibodies (monoclonal antibodies, polyclonal antibodies) and their fragments (F(ab')₂, Fab', Fab, F(abc'), Fabc' and the like), various receptors, various avidins (avidin D, streptavidin and the like), protein A, protein G, protein L, various lectins (concanavalin A, lentil lectin, phytohaemagglutinin and the like), probes capable of binding to each nucleic acid to be analyzed and the like can be used as a probe.

Any antibodies that bind to target antigens or materials to be analyzed may be used. Fragments of an antibody such as F(ab')₂ and Fab can be obtained from the antibody by using protease such as pepsin and papain. The heavy chains (H chain) of an antibody are generally linked to each other via S-S bonds, and the bonds can be broken by a reducing agent. The reducing agents include cysteamine and mercaptoethanol. F(ab')₂ is cleaved into Fab' by such a reducing agent to newly generate thiol (SH) groups. These fragments of antibody such as F(ab')₂, Fab', Fab, F(abc'), Fabc' and the like can be also used in the present invention.

To describe the present invention in detail and in a non-limiting way, examples of the process for production of the enzyme-probe complex and their characteristics will be shown below.

### Example 1

### Preparation of enzyme-anti HCV core antigen monoclonal antibody complex using Dextran T2000 as a carrier

0.3 g of Dextran T2000 (average molecular weight: 2,000,000; Amersham) was weighed and dissolved in 6 ml of 0.1 M phosphate buffer (pH 7.0). 3 ml of sodium periodate solution was added and mixed, and the mixture was left standing at room temperature for 2 hours to react, and then subjected to gel filtration (Sephadex G25, Amersham) to collect the void fraction. Hydrazine HCl (Wako Pure Chemical Industries Inc.) was added to the void fraction to introduce hydrazine to dextran. In theory, the maximum 22,000 hydrazine can be introduced to a dextran molecule with a molecular weight of 2,000,000. 100 mg of peroxidase (HRP, Toyobo Co. Ltd.) was weighed, mixed and dissolved in 3 ml of 0.1 M sodium bicarbonate solution. 1.5 ml of sodium periodate solution was added and mixed, and the mixture was left standing at room temperature for 2 hours to react. The mixture was desalted by gel filtration (Sephadex G25) and, then added and mixed with about 150 mg of dextranhydrazide obtained in advance. The mixture was reacted at room temperature for 2 hours, and after reduction, glycine was added to a final concentration of 0.1 M. The mixture was dialyzed 3 times at 4°C for 4 hours each to obtain the blocked carrier HRP conjugate. Since unbound HRP was 10% or less in this reaction, also in view of recovery yield, it would appear that about 12 to 16 HRP are bound to one molecule of Dextran T2000 (average molecular weight: 2,000,000). 1 mg of N-(6-maleimidecaproyloxy)succinimide (EMCS, Dojindo Laboratories) dissolved in dimethylformamide (DMF) was added to 5 mg of the conjugate and reacted at room temperature for 2 hours, and then excess EMCS was removed by gel filtration (Sephadex G 25) to introduce maleimide to the carrier HRP conjugate. To 3.5 mg of F(ab')₂ of anti-HCV core antigen monoclonal antibodies consisting of an equal amount of c11-10F (ab')₂ and c11-14F(ab')₂ in 0.1 M phosphate buffer (pH 6.0), 1/10 volume of 0.1 M cysteamine HCl solution was added and the mixture was incubated at 37°C for 90 minutes to convert the F(ab')₂ to Fab'. The mixture was subjected to gel filtration (Sephadex G25), and Fab' was obtained by collecting the void fraction. This Fab' and the carrier HRP conjugate, to which maleimide groups were introduced, were reacted at 4°C overnight and subjected to gel filtration (Superdex 200 pg, 1.6 × 60 cm, Amersham) to remove free Fab'. At this time, the HRP-Fab complex of the present invention is eluted near the void fractions. Since free Fab' was 10% or less in this reaction, also in view of the recovery yield, it would appear that about 6 to 10 Fab' are bound to one molecule of Dextran T2000 (average molecular weight: 2,000,000). Bovine serum albumin (BSA) was added to the collected fractions to 0.5% and the mixture was stored at 4°C. Absorbance of the HRP-Fab complex thus prepared was measured at 403 nm and the concentration of HRP in the complex was calculated using the molecular absorption coefficient of HRP at 403 nm.

### Example 2

### Preparation of enzyme-anti HCV core antigen monoclonal antibody complex using Dextran T70 as a carrier

0.3 g of Dextran T70 (average molecular weight: 70,000; Amersham) was weighed and HRP-Fab complex was obtained in the similar manner to Example 1. Absorbance of the HRP-Fab complex was measured at 403 nm and the concentration of HRP in the complex was calculated using the molecular absorption coefficient of HRP at 403 nm.

### Example 3

### Preparation of enzyme-anti HCV core antigen monoclonal antibody complex using Dextran T500 as a carrier by changing ratio of carrier to enzyme

0.3 g of Dextran T500 (average molecular weight: 500,000; Amersham) was weighed, and the carrier-enzyme conjugate was prepared: at weight ratio (0.66) of carrier (150 mg): enzyme (100 mg), which is similar to Example 1; at a weight ratio (2.00) of carrier 150 mg: 2 times amounts of enzyme (300 mg); and at a weight ratio (3.33) of carrier 150 mg: 3.3 times amounts of enzyme (500 mg). After that anti-HCV core antigen monoclonal antibody was conjugated in the similar manner to Example 1. Absorbance of the HRP-Fab complex was measured at 403 nm and the concentration of HRP in the complex was calculated using the molecular absorption coefficient of HRP at 403 nm.

### Example 4

### Preparation of enzyme-anti-ProGRP antibody complex using Dextran T500 as a carrier

0.3 g of Dextran T500 (average molecular weight: 500,000; Amersham) was weighed, and the carrier-HRP conjugate was obtained in the similar manner to Example 1. To 5 mg of this conjugate, 1 mg of EMCS dissolved in DMF was added and reacted at room temperature for 2 hours. Excess EMCS was removed by gel filtration (Sephadex G25) and maleimide was introduced to the carrier HRP conjugate. To the solution of F(ab')₂ of anti-ProGRP monoclonal antibody (2B10) in 0.1 M phosphate buffer (pH 6.0), 1/10 volume of 0.1 M cysteamine hydrochloride was added and the mixture was incubated at 37°C for 90 minutes to produce Fab'. The mixture was subjected to gel filtration (Sephadex G25) and the void fractions were collected to obtaion Fab'. This Fab' and the carrier HRP conjugate, to which maleimide was introduced, were reacted at 4°C overnight and subjected to gel filtration (Superdex 200 pg, 1.6 × 60 cm, Amersham). Fractions near the void were collected, mixed with bovine serum albumin (BSA) to a final concentration of 0.5% and stored at 4°C. Absorbance of the HRP-Fab complex thus prepared was measured at 403 nm and the concentration of HRP in the complex was calculated using the molecular absorption coefficient of HRP at 403 nm.

### <Comparative Example 1>

### Preparation of enzyme-anti-HCV core antigen monoclonal antibody using conventional method

The method for preparing labeled antibody reported by Ishikawa et al., has been widely applied to general diagnostic reagents. This method (Ultra high sensitive enzyme immunoassay method, Eiji Ishikawa, 1993, Japan Scientific Societies Press) was used as the conventional method. 4 mg of HRP was weighed and dissolved in 0.6 ml of 0.1 M phosphate buffer (pH 7.0). 1 mg of EMCS dissolved in DMF was added and reacted at room temperature for 2 hours. Excess EMCS was removed by gel filtration (Sephadex G25) and maleimide was introduced to amino groups of HRP. To each solution of F(ab')₂ of anti-HCV core antigen monoclonal antibody (c11-10 and c11-14) in 0.1 M phosphate buffer (pH 6.0), 1/10 volume of 0.1 M cysteamine hydrochloride solution was added and the mixture was incubated at 37°C for 90 minutes to produce Fab'. Fab' was subjected to gel filtration (Sephadex G25) and the void fraction was collected. This Fab' and HRP, to which maleimide was introduced, were reacted at 4°C overnight, and the mixture was subjected to gel filtration (Superdex 200pg, 1.6 × 60 cm), and the HRP-Fab fraction was collected, mixed with bovine serum albumin (BSA) to a final concentration of 0.5% and stored at 4°C. Absorbance of the HRP-Fab conjugate thus prepared was measured at 403 nm and the concentration of HRP in the conjugate was calculated using the molecular absorption coefficient of HRP at 403 nm.

### <Comparative Example 2>

### Preparation of enzyme-anti-ProGRP monoclonal antibody using conventional method

Maleimide was introduced to amino groups in HRP in the similar manner to Comparative Example 1. To the solution of F(ab')₂ of anti-ProGRP monoclonal antibody (2B10) in 0.1 M phosphate buffer (pH 6.0), 1/10 volume of 0.1 M cysteamine hydrochloride was added and the mixture was incubated at 37°C for 90 minutes to produce Fab'. Fab' was subjected to gel filtration (Sephadex G25) and the void fraction was collected. This Fab' and HRP, to which maleimide was introduced, were reacted at 4°C overnight, and the mixture was subjected to gel filtration (Superdex 200pg, 1.6 × 60 cm), and the HRP-Fab fraction was collected, mixed with bovine serum albumin (BSA) to a final concentration of 5% and stored at 4°C. Absorbance of the HRP-Fab conjugate thus prepared was measured at 403 nm and the concentration of HRP in the conjugate was calculated using the molecular absorption coefficient of HRP at 403 nm.

### Example 5

### Molecular weight analysis by gel filtration for enzyme-anti-HCV core antigen monoclonal antibody complex prepared in Example 1

The enzyme-anti-HCV core antigen monoclonal antibody complex prepared in Example 1 was subjected to gel filtration using Sephacryl S-500 Superfine (amersham) (1.6 × 60) column. This Sephacryl S-500 Superfine column is used mainly for separating molecules with a large molecular weight and small particles. According to "Gel filtration theory and practice" by Pharmacia, the fractionation range of this column is 4 × 10⁴ to 2 × 10⁷ for polysaccharides. Gel filtration was performed using PBS as a carrier at a flow rate of 1 ml/min. 4 ml/2 min fractions were collected, absorbance at 403 nm was measured and HRP concentration (µg/ml) was calculated using the molecular absorption coefficient of HRP. Each fraction was diluted to 1 µg/ml equivalent of HRP concentration and the reactivity was investigated. The method for assay is as follows.

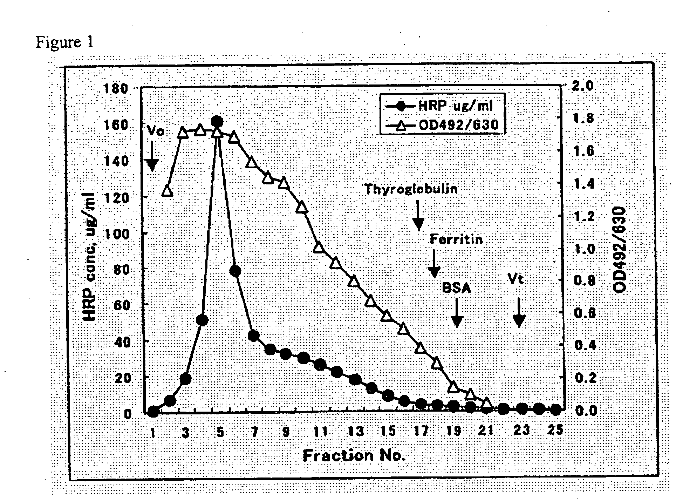

To each well of a 96 well microtiter plate, 200 µl of anti-HCV core antigen monoclonal antibody (mixture of an equal amount of c11 -3 and c11-7) at a concentration of 4µg/ml was added and incubated at 4°C overnight. After washing with 10 mM phosphate buffer (pH 7.3), 350 µl of 0.5% casein was added to each well and incubated for 2 hours. Samples in which concentration of recombinant HCV core antigen (c11) was adjusted at 0 fmol/L and 1200 fmol/L were added and incubated at room temperature for 60 minutes while shaking. After washing 6 times with 10 mM phosphate buffer (pH 7.3) containing 0.05% Tween 20 (washing solution), 200 µl of each fraction, which was diluted to 1 µg/ml of HRP equivalent concentration, and conventionally prepared enzyme-antibody conjugate at 1 µg/ml of HRP equivalent concentration were added as the secondary antibody and incubated for 30 minutes. Each well was further washed 6 times with washing solution, and 200 µl of substrate solution (ortho-phenylenediamine, hydrogen peroxide) was added and incubated for 30 minutes. The enzyme reaction was terminated by adding 50 µl of 5N sulfuric acid, and absorbance at 492 nm (reference wavelength: 630 nm) was measured by using a microtiter plate reader (Corona MTP32). Table 1 and Fig 1 show the HRP concentration of each gel filtration fraction and the results of the above mentioned assay (difference of the absorbance between core antigen 1200 fmol/L and 0 fmol/L). Molecular weight of 3 molecular weight markers are: Thyroglobulin, 668,000; Ferritin, 440,000; and BSA, 68,000. In addition, measured at the same time, absorbance of 1 µg/ml of the enzyme-antibody conjugate prepared by the conventional method at core antigen 0 fmol/L and 1200 fmol/L was 0.000 and 0.050, respectively. Thus, the difference between absorbance of 1200 fmol/L and 0 fmol/L of core antigen by the conventional method was 0.050. Figure 1 confirms that the larger the molecular weight is, the higher the absorbance of core antigen at 1200 fmol/L becomes.

**[Table 1]**

| Fraction No. | HRP µg/ml | OD492/630 | Ratio of absorbance of each fraction to absorbance of conventional method (0.050) |
|---|---|---|---|
| 1 | 0.6 | | |
| 2 | 6.3 | 1.372 | 27.4 |
| 3 | 18.7 | 1.724 | 34.5 |
| 4 | 51.3 | 1.736 | 34.7 |
| 5 | 160.7 | 1.724 | 34.5 |
| 6 | 78.4 | 1.894 | 33.9 |
| 7 | 42.0 | 1.539 | 30.8 |
| 8 | 34.5 | 1.446 | 28.9 |
| 9 | 31.7 | 1.413 | 28.3 |
| 10 | 29.3 | 1.266 | 25.3 |
| 11 | 25.8 | 1.016 | 20.3 |
| 12 | 21.8 | 0.918 | 18.4 |
| 13 | 17.3 | 0.801 | 16.0 |
| 14 | 12.6 | 0.682 | 13.6 |
| 15 | 8.5 | 0.589 | 11.8 |
| 16 | 5.5 | 0.511 | 10.2 |
| 17 | 3.9 | 0.387 | 7.7 |
| 18 | 3.0 | 0.298 | 5.9 |
| 19 | 2.5 | 0.147 | 2.9 |
| 20 | 1.6 | 0.102 | 2.0 |
| 21 | 1.1 | 0.049 | 1.0 |
| 22 | 0.5 | | |
| 23 | 0.4 | | |
| 24 | 0.4 | | |
| 25 | 0.0 | | |

In fractions after 19, the increase of the signal compared to the conventional method was 3 fold or less, but in fractions 2 to 18, the increase of the signal was 3 fold or more. Since thyroglobulin (molecular weight 668,000) and Ferritin (molecular weight 440,000) showed the peak near fraction 17 and fraction 18, respectively, it was confirmed that the present invention demonstrates about 5.9 fold or more superior effect than the conventional method at the molecular weight of about 440,000 or above and about 7.7 fold superior effect at the molecular weight of 668,000 or above.

The average molecular weight of Dextran T2000 in the present Examples is 2,000,000, and when 14 molecules of HRP with a molecular weight of 40,000 are bound to this carrier, and further 8 molecules of Fab' with a molecular weight of 46,000 are bound to this blocked carrier-enzyme, the molecular weight of the enzyme-probe complex is about 2,900,000. This is to be understood as the basic unit of the enzyme-probe complex in the present Examples.

On the other hand, the fractionation range of Sephacryl S-500 Superfine column which was used for gel filtration in the present Examples is 4 × 10⁴ to 2 × 10⁷, and as clearly shown in Table 1, the first fraction including the void fraction shows a high activity. The first fraction including the void fraction contains the enzyme-probe complex with a molecular weight of at least 2 × 10⁷ or above. Since the molecular weight of the basic unit of the enzyme-probe complex in the present Example is 2,900,000, it can be interpreted that the enzyme-probe complex present in the first fraction including the void fraction forms an enzyme-probe complex having the larger molecular weight in which at least two or more enzyme-probe complexes are covalently joined, that is the blocked enzyme-probe complex.

Further, the carrier Dextran T2000 used in the present Example must contain Dextran with a molecular weight less than the average molecular weight of 2,000,000 and their degraded products. It is obvious that these are also involved in the formation of the enzyme-probe complex or blocked enzyme-probe complex, and thus in the present Examples the enzyme-probe complex with a molecular weight of less than 2,900,000 is included which is the average molecular weight of the basic unit of the enzyme-probe complex. These are also included in the present invention.

### Example 6

### Reactivity of enzyme-anti-HCV core antigen monoclonal antibody complexes when ratio of carrier to enzyme is changed

Using the enzyme-anti-HCV core antigen monoclonal antibody complexes prepared in Example 3, the reactivity of these complexes were studied after diluting to 2 µg/ml equivalent enzyme concentration. The following is the test method.

To each well of a 96 well microtiter plate, 200 µl of anti-HCV core antigen monoclonal antibody (mixture of an equal amount of c11-3 and c11 -7) at a concentration of 4 µg/ml was added and incubated at 4°C overnight. After washing with 10 mM phosphate buffer (pH 7.3), 350 µl of 0.5% casein was added to each well and incubated for 2 hours. Concentration of recombinant HCV core antigen (c11) was adjusted to 0 fmol/L, 25.9 fmol/L, 77.8 fmol/L, 233.3 fmol/L and 700 fmol/L, added as samples and incubated at room temperature for 60 minutes while shaking. After washing 6 times with 10 mM phosphate buffer (pH 7.3) containing 0.05% Tween 20, each enzyme-anti-HCV core antigen monoclonal antibody complex, which was diluted to 2 µg/ml enzyme equivalent concentration and incubated for 30 minutes. Each well was further washed 6 times with washing solution, and 200 µl of substrate solution (ortho-phenylenediamine, hydrogen peroxide) was added and incubated for 30 minutes. The enzyme reaction was terminated by adding 50 µl of 5N sulfuric acid, and absorbance at 492 nm (reference wavelength: 630 nm) was measured using a microtiter plate reader (Corona MTP32).

In comparison of reactivities of anti-HCV core antigen monoclonal antibody complexes prepared by using carrier-enzyme conjugate, in which the weight ratios of carrier to enzyme are 0.66, 2.00 and 3.33, the reactivities of the complexes with the weight ratio of 2.00 and 3.33 were 77.6% and 70.5% of the reactivity of the complex with the weigh ratio of 0.66, indicating that the more the amount of the enzyme, the lower the reactivity is. Even the complexes with the carrier: enzyme weight ratio of 2.00, 3.33 or more still demonstrate sufficiently higher sensitivities than the conventional ones. However, when excess amount of enzyme exists over the carrier, many more enzyme-carriers, which are the minimum unit, are produced because many more enzyme bind to one carrier molecule. However, it is speculated that there is less chance of producing complexes in which the minimum units are bind to each other via enzyme, such as carrier-enzyme-carrier-enzyme-carrier-enzyme. That is, as described in the gel filtration analysis in Example 5, it can be interpreted that the reactivity of the enzyme-probe complex of the present invention is higher because an enzyme-probe complex having the larger molecular weight in which at least two or more enzyme-probe complexes as the minimum unit of the complex are covalently joined is formed, in other words because the blocked enzyme-probe complex is formed.

### Example 7

### Comparison of sensitivity for core antigen detection, using enzyme-anti-HCV core antigen monoclonal antibody complex prepared in Example 1 and 2, and conventional enzyme-antibody

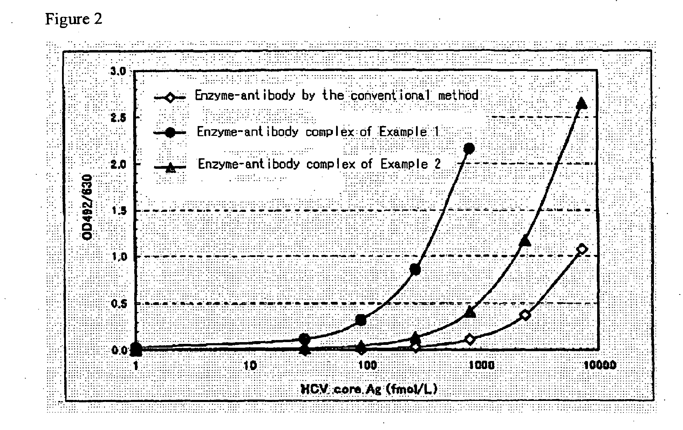

To each well of a 96 well microtiter plate, 200 µl of anti-HCV core antigen monoclonal antibody (mixture of an equal amount of c11-3 and c11-7) at a concentration of 4 µg/ml was added and incubated at 4°C overnight. After washing with 10 mM phosphate buffer pH 7.3 (PBS), 350 µl of 0.5% casein was added to each well and incubated for 2 hours. After removing 0.5% casein by suction, recombinant HCV core antigen (c11) was serially diluted by 3 fold from 21870 fmol/L, added as samples and incubated at room temperature for 60 minutes while shaking. After washing 6 times with washing solution, 200 µl of the enzyme-anti HCV core antigen monoclonal antibody complex prepared in Examples 1 and 2, or enzyme-antibody prepared by the conventional method was added as the secondary antibody at 2.5 µg/ml of HRP and incubated for 30 minutes. Each well was further washed 6 times with washing solution, and 200 µl of substrate solution (10 mg of ortho-phenylenediamine, hydrogen peroxide) was added and incubated for 30 minutes. The enzyme reaction was terminated by adding 50 µl of 5N sulfuric acid, and absorbance at 492 nm (reference wavelength: 630 nm) was measured using a microtiter plate reader (Corona MTP32). Figure 2 shows the result. If the difference between 0 fmol/L and OD 0.030 is assumed to be the detection limit, conventional enzyme-antibody, enzyme-antibody complex of Example 2 and enzyme-antibody complex of Example 1 can detect about 263 fmol/L, about 75 fmol/L and about 10 fmol/L (0.2 pg/ml), respectively. That is, the sensitivity of the present invention is higher compared to the conventional method, by 3. 5 to 26.3 fold. With higher sensitivity, the utility of the method is improved because of the ease determination of HCV infection and the wide range of virus quantification.

### Example 8

### Comparison of sensitivity for core antigen detection, using enzyme-anti-HCV core antigen monoclonal antibody complex in which 2 kinds of monoclonal antibody are bound together and enzyme-anti-HCV core antigen monoclonal antibody complex in which 2 kinds of monoclonal antibody are separately bound

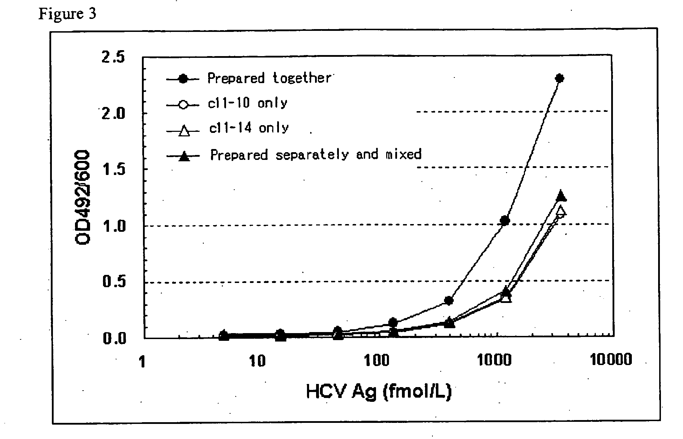

In Example 1 or 2, two kinds of anti-HCV core antigen monoclonal antibody {c11-10F(ab')₂ and c11 -14F(ab')₂} were reacted together to the blocked carrier-enzyme. In addition, these two kinds of monoclonal antibody were reacted separately with the blocked carrier-enzyme and the enzyme-anti-HCV core antigen monoclonal antibody complexes were prepared. Reactivity was studied for the preparation in which two kinds of monoclonal antibody was bound together and for the preparations in which c11-10 and c11-14 monoclonal antibody was bound singly at a concentration of 1 µg/ml. Reactivity was also studied by mixing the preparation of singly bound c11 -10 monoclonal antibody and the preparation of singly bound c11-14 monoclonal antibody, each at 0.5 µg/ml (total 1 µg/ml). Results are shown in Figure 3.

To each well of a 96 well microtiter plate, 200 µl of anti-HCV core antigen monoclonal antibody (mixture of an equal amount of c11-3 and c11-7) at a concentration of 4 µg/ml was added and incubated at 4°C overnight. After washing with 10 mM phosphate buffer pH 7.3 (PBS), 350 µl of 0.5% casein was added to each well and incubated for 2 hours. After removing 0.5% casein by suction, recombinant HCV core antigen (c11) was serially diluted by 3 fold from 21870 fmol/L, added as samples and incubated at room temperature for 60 minutes while shaking. After washing 6 times with washing solution, 200 µl at 1 µg/ml HRP concentration of the preparation in which two kinds of monoclonal antibody were bound together, the preparations in which c11 -10 and c11-14 monoclonal antibodies were bound singly and the mixture of an equal amount of singly bound c 11-10 monoclonal antibody preparation and c11-14 monoclonal antibody preparation were added and incubated for 20 minutes. Each well was further washed 6 times with washing solution, and 200 µl of substrate solution (10 mg of ortho-phenylenediamine, hydrogen peroxide) was added and incubated for 30 minutes. The enzyme reaction was terminated by adding 50 µl of 5N sulfuric acid, and absorbance at 492 nm (reference wavelength: 630 nm) was measured using a microtiter plate reader (Corona MTP32).

Reactivity was a little improved (about 1.2 fold) in the mixture of 0.5 µg/ml each of c11-10 and c11-14 monoclonal antibody singly bound than either one of them alone. However, the reactivity of the preparation with two kinds of antibody bound together was increased about 2 to 2.5 fold over the mixed preparation. Thus it is more effective to bind the probe such that two or more kinds of monoclonal antibody and the fragment thereof to the blocked carrier enzyme.

### Example 9

### Reactivity of enzyme-anti-HCV core antigen monoclonal antibody complex in which monoclonal antibody is bound specifically to the carrier on the blocked carrier-enzyme

The number of amino groups in HRP is very few, and the results of Ishikawa et al. who tried to introduce maleimide groups to HRP using amino groups indicate that there are at most 1 to 3 groups (Eiji Ishikawa,; Methods for Biochemistry Experiments 27, Enzyme Labeling Method, Japan Scientific Societies Press). After blocking these few amino groups in HRP using 2-methylmaleic anhydride, a carrier-enzyme conjugate was prepared using Dextran T500 as a carrier, at a carrier: enzyme weight ratio of 0.66 in the similar manner to Example 3, and then anti-HCV core antigen monoclonal antibody was bound. Absorbance at 403 nm was measured and HRP concentration in the complex was calculated using the molecular absorption coefficient of HRP at 403 nm.

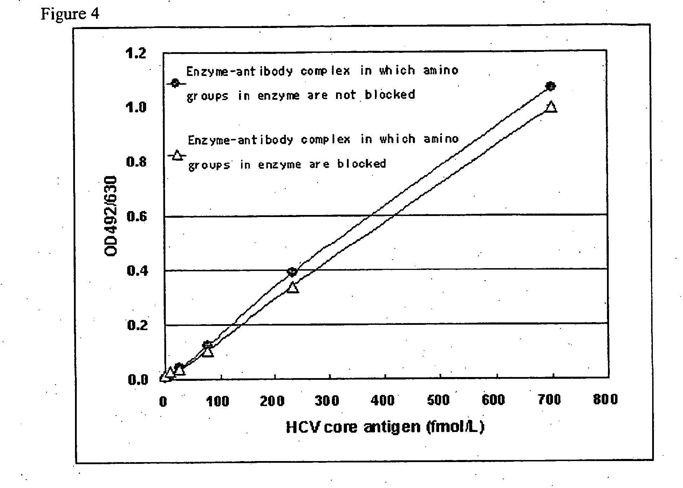

To each well of a 96 well microtiter plate, 200 µl of anti-HCV core antigen monoclonal antibody (mixture of an equal amount of c11-3 and c1 1-7) at a concentration of 4 µg/ml was added and incubated at 4°C overnight. After washing with 10 mM phosphate buffer pH 7.3 (PBS), 350 µl of 0.5% casein was added to each well and incubated for 2 hours. After removing 0.5% casein by suction, recombinant HCV core antigen (c11) was serially diluted by 3 fold from 21870 fmol/L, added as samples and incubated at room temperature for 60 minutes while shaking. After washing 6 times with washing solution, 200 µl of labeled antibody was added at a concentration of total 2 µg/ml equivalent of HRP concentration and incubated for 20 minutes. Each well was further washed 6 times with washing solution, and 200 µl of substrate solution (10 mg of ortho-phenylenediamine, hydrogen peroxide) was added and incubated for 30 minutes. The enzyme reaction was terminated by adding 50 µl of 5N sulfuric acid, and absorbance at 492 nm (reference wavelength: 630 nm) was measured using a microtiter plate reader (Corona MTP32). As a comparative reference, carrier-enzyme conjugate was prepared at a carrier: enzyme weight ratio 0.66 in the similar manner to Example 3, and then used after binding anti-HCV core antigen monoclonal antibody (Figure 4).

As shown in Figure 4, the reactivity of the enzyme-antibody complex in which amino groups were blocked demonstrated about 88.1% activity compared with that without block and thus it was confirmed that sufficient reactivity was obtained when antibody was bound to only the carrier. Since a large excess of enzyme was not used in the blocked enzyme-antibody complex of the present invention, antibody can bind to either carrier or enzyme but it seems that sufficient reactivity is shown when the antibody that is a probe is bound to only the carrier.

### Example 10

### Comparison of detection sensitivity using enzyme-anti-ProGRP monoclonal antibody complex prepared in Example 4 and conventional enzyme-antibody

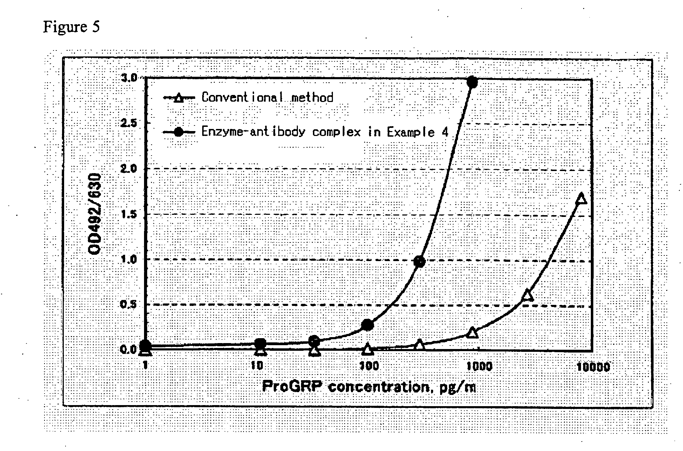

To each well of a 96 well microtiter plate, 200 µl of anti-ProGRP monoclonal antibody (2B10) at a concentration of 5 µg/ml was added and incubated at 4°C overnight. After washing twice with 10 mM phosphate buffer pH 7.3 (PBS), 350 µl of 0.5% casein was added to each well and incubated for 2 hours. After removing 0.5% casein by suction, recombinant ProGRP (31-98) was serially diluted by 3 fold from 8000 pg/ml, added as samples and incubated at 37°C for 60 minutes. After washing 5 times with washing solution, 200 µl of the enzyme-anti-ProGRP monoclonal antibody complex prepared in Example 4 or enzyme-antibody prepared by the conventional method was added as the secondary antibody at a concentration of total 1.5 µg/ml of HRP and incubated for 30 minutes. Each well was further washed 6 times with washing solution, and 200 µl of substrate solution (10 mg of ortho-phenylenediamine, hydrogen peroxide) was added and incubated for 30 minutes. The enzyme reaction was terminated by adding 50 µl of 5N sulfuric acid, and absorbance at 492 nm (reference wavelength: 630 nm) was measured using a microtiter plate reader (Corona MTP32). The results are shown in Figure 5. The horizontal axis represents concentration of ProGRP and the vertical axis represents absorbance at 492 nm. It is clearly seen that the enzyme antibody complex prepared in Example 4 shows extremely high signal compared to the enzyme-labeled antibody prepared by the conventional method. Assuming that the difference between 0 pg/ml and OD 0.020 is the detection limit, the conventional enzyme-antibody and the enzyme-antibody complex of Example 4 can detect about 115 pg/ml and about 7 pg/ml, respectively. That is, the sensitivity of the method of the present invention is 16.4 fold higher compared to the conventional method. Since the concentration of ProGRP in serum of healthy individuals is about 14 pg/ml and the cut-off value is about 50 pg/ml (Jpn. J. Cancer Res. 1995, 86, 698-705), ProGRP cannot be detected in specimens from healthy individuals or some of the patients of small cell lung cancer by the conventional method. By the present invention, it became possible to detect ProGRP in specimens from patients and healthy individuals, which the conventional method could not detect, and thus confirming the usefulness of the present invention.

## Claims

1. A blocked enzyme-probe complex wherein a probe molecule is conjugated to a complex in which two or more molecules as carrier having a molecular weight of 20,000 to 4,000,000 Da are linked via an enzyme bound to said carrier, the carrier being selected from the group consisting of polysaccharides, high molecular weight proteins and peptide polymers wherein more enzyme molecules are bound to the complex.

2. The blocked enzyme-probe complex according to claim 1 wherein the carrier and the enzyme molecule are bound through a functional group on the carrier and an aldehyde group formed by oxidizing a carbohydrate chain within the enzyme molecule.

3. The blocked enzyme-probe complex according to any one of claim 1 or 2 wherein the carrier is one or more varieties selected from the group consisting of dextran, aminodextran, ficoll, dextrin, agarose, pullulan, various celluloses, chitin, chitosan, β-galactosidase, thyroglobulin, hemocyanin, polylysine, polypeptide and DNA.

4. The blocked enzyme-probe complex according to any one of claim 1 or 2 wherein the enzyme is one or more varieties selected from the group consisting of horseradish peroxidase, alkaline phosphatase, β-galactosidase, glucose oxidase and luciferase.

5. The blocked enzyme-probe complex according to any one of claim 1 or 2 wherein the probe is one or more varieties selected from the group consisting of an antibody molecule or a functional fragment thereof, protein A, protein G, protein L, lectin, a receptor and avidin.

6. The blocked enzyme-probe complex according to any one of claims 1 to 5 wherein two or more kinds of probe are conjugated.

7. The blocked enzyme-probe complex according to claim 5 or 6 wherein the antibody molecule or the functional fragment thereof is one or more varieties selected from the group consisting of anti-HCV core antigen antibody, anti-gastrin releasing peptide precursor antibody and the functional fragments thereof.

8. The blocked enzyme-probe complex according to any one of claims 1 to 7 wherein the molecular weight of the blocked enzyme-probe complex is 440,000 Da or more.

9. An immunoassay kit or a nucleic acid detection reagent comprising the blocked enzyme-probe complex according to any one of claims 1 to 8.

10. A method for producing the blocked enzyme probe complex according to any one of claims 1 to 8 comprising the steps of forming a blocked material by binding a carrier having a molecular weight of 20,000 to 4,000,000 Da to an enzyme; and conjugating a probe to the blocked material.

11. The method according to claim 10 wherein the blocked material is formed by reacting the carrier and the enzyme molecule at a weight ratio of carrier:enzyme = 1:0.1 to 1:20.

## Patentansprüche

1. Blockierter Enzym-Sondenkomplex, worin ein Sondenmolekül an einen Komplex konjugier ist, in welchem zwei oder mehrere Moleküle als Träger mit einem Molekulargewicht von 20.000 bis 4.000.000 Da über eine Enzymbindung an den Träger gebunden sind, wobei der Träger aus der Gruppe gewählt ist, bestehend aus Polysacchariden, hochmolekulargewichtigen Proteinen und Peptidpolymeren, wobei mehrere Enzymmoleküle an den Komplex gebunden sind.

2. Blockierter Enzym-Sondenkomplex nach Anspruch 1, wobei der Träger und das Enzymmolekül über eine funktionelle Gruppe auf dem Träger und eine durch Oxidation einer Kohlenhydratkette innerhalb des Enzymmoleküls gebildete Aldehydgruppe gebunden sind.

3. Blockierter Enzym-Sondenkomplex nach irgendeinem der Ansprüche 1 oder 2, wobei der Träger eine oder mehrere Varietäten ist, gewählt aus der Gruppe, bestehend aus Dextran, Aminodextran, Ficoll, Dextrin, Agarose, Pullulan, verschiedene Cellulosen, Chitin, Chitosan, β-Galactosidase, Thyroglobulin, Hemocyanin, Polylysin, Polypeptid und DNA.

4. Blockierter Enzym-Sondenkomplex nach irgendeinem der Ansprüche 1 oder 2, wobei das Enzym eine oder mehrere Varietäten ist, gewählt aus der Gruppe, bestehend aus Meerrettich-Peroxidase, alkalische Phosphatase, β-Galactosidase, Glucoseoxidase und Luciferase.

5. Blockierter Enzym-Sondenkomplex nach irgendeinem der Ansprüche 1 oder 2, wobei die Sonde eine oder mehrere Varietäten ist, gewählt aus der Gruppe, bestehend aus einem Antikörpermolekül oder einem funktionellen Fragment davon, Protein A, Protein G, Protein L, Lectin, einem Rezeptor und Avidin.

6. Blockierter Enzym-Sondenkomplex nach irgendeinem der Ansprüche 1 bis 5, wobei die zwei oder mehrere Arten der Sonde konjugiert sind.

7. Blockierter Enzym-Sondenkomplex nach Anspruch 5 oder 6, wobei das Antikörpermolekül oder das funktionelle Fragment davon eine oder mehrere Varietäten ist, gewählt aus der Gruppe, bestehend aus Anti-HCV-Kernantigen-Antikörper, Anti-Gastrin freisetzender Peptidvorläufer-Antikörper und funktionellen Fragmenten davon.

8. Blockierter Enzym-Sondenkomplex nach irgendeinem der Ansprüche 1 bis 7, wobei das Molekulargewicht des blockierten Enzym-Sondenkomplexes 440.000 Da oder mehr beträgt.

9. Immunoassay-Kit oder ein Nucleinsäure-Detektionsreagens, umfassend den blockierten Enzym-Sondenkomplex nach irgendeinem der Ansprüche 1 bis 8.

10. Verfahren zur Herstellung des blockierten Enzym-Sondenkomplexes nach irgendeinem der Ansprüche 1 bis 8, umfassend die Schritte des Bildens eines blockierten Materials durch Binden eines Trägers mit einem Molekulargewicht von 20.000 bis 4.000.000 Da an ein Enzym; und Konjugieren einer Sonde mit dem blockierten Material.

11. Verfahren nach Anspruch 10, wobei das blockierte Material gebildet wird durch Umsetzen des Trägers und des Enzymmoleküls bei einem Gewichtsverhältnis von Träger : Enzym = 1:0,1 bis 1:20.

## Revendications

1. Complexe bloqué enzyme-sonde, dans lequel une molécule de sonde est conjuguée à un complexe dans lequel deux ou plus de deux molécules servant de support ayant un poids moléculaire de 20 000 à 4 000 000 Da sont liées par une enzyme liée audit support, le support étant choisi dans le groupe consistant en des polysaccharides, des protéines de haut poids moléculaire et des polymères peptidiques, plusieurs molécules de l'enzyme étant liées au complexe.

2. Complexe bloqué enzyme-sonde suivant la revendication 1, dans lequel le support et la molécule d'enzyme sont liés par un groupe fonctionnel sur le support et un groupe aldéhyde formé par oxydation d'une chaîne glucidique dans la molécule d'enzyme.

3. Complexe bloqué enzyme-sonde suivant l'une quelconque des revendications 1 et 2, dans lequel le support consiste en une ou plusieurs variétés choisies dans le groupe consistant en le dextrane, l'aminodextrane, le Ficoll, la dextrine, l'agarose, le pullulane, diverses celluloses, la chitine, le chitosane, la β-galactosidase, la thyroglobuline, l'hémocyanine, la polylysine, un polypeptide et un ADN.

4. Complexe bloqué enzyme-sonde suivant l'une quelconque des revendications 1 et 2, dans lequel l'enzyme consiste en une ou plusieurs variétés choisies dans le groupe consistant en la peroxydase de raifort, la phosphatase alcaline, la β-galactosidase, la glucose-oxydase et la luciférase.

5. Complexe bloqué enzyme-sonde suivant l'une quelconque des revendications 1 et 2, dans lequel la sonde consiste en une ou plusieurs variétés choisies dans le groupe consistant en une molécule d'anticorps ou un de ses fragments fonctionnels, la protéine A, la protéine G, la protéine L, une lectine, un récepteur et l'avidine.

6. Complexe bloqué enzyme-sonde suivant l'une quelconque des revendications 1 à 5, dans lequel deux ou plus de deux types de sondes sont conjugués.

7. Complexe bloqué enzyme-sonde suivant la revendication 5 ou 6, dans lequel la molécule d'anticorps ou son fragment fonctionnel consistent en une plusieurs variétés choisies dans le groupe consistant en un anticorps anti-antigène nucléocapsidique du HCV, un anticorps anti-précurseur du peptide de libération de gastrine et leurs fragments fonctionnels.

8. Complexe bloqué enzyme-sonde suivant l'une quelconque des revendications 1 à 7, dans lequel le poids moléculaire du complexe bloqué enzyme-sonde est égal ou supérieur à 440 000 Da.

9. Kit d'analyse immunologique ou réactif de détection d'acide nucléique, comprenant le complexe bloqué enzyme-sonde suivant l'une quelconque des revendications 1 à 8.

10. Procédé pour la production du complexe bloqué enzyme-sonde suivant l'une quelconque des revendications 1 à 8, comprenant les étapes de formation d'une matière bloquée par liaison d'un support ayant un poids moléculaire de 20 000 à 4 000 000 Da à une enzyme ; et de conjugaison d'une sonde à la matière bloquée.

11. Procédé suivant la revendication 10, dans lequel la matière bloquée est formée par réaction du support et de la molécule d'enzyme en un rapport pondéral support:enzyme de 1:0,1 à 1:20.
